# EUROPEAN PATENT SPECIFICATION

(11) **EP 1 006 963 B1**
(45) Date of publication and mention of the grant of the patent: **07.04.2004**
(21) Application number: 97950949.4
(22) Date of filing: 05.12.1997
(51) Int. Cl.: A61F 5/08

(54) **THERMAL NASAL DILATOR**
THERMISCHER NASALER DILATATOR
DILATATEUR NASAL THERMIQUE

(30) Priority: 20.12.1996 US 771192
(43) Date of publication of application: 14.06.2000
(73) Proprietor: THE PROCTER & GAMBLE COMPANY, Cincinnati, Ohio 45202 (US)
(72) Inventor: MITRA, Sekhar, Cincinnati, OH 45243 (US)
(74) Representative: Clemo, Nicholas Graham
(86) International application number: PCT/US1997/022946
(87) International publication number: WO 1998/027897

(56) References cited:
- WO-A-93/19706
- DE-A- 3 416 146
- US-A- 4 108 146
- US-A- 4 749 700
- US-A- 5 533 503

## Description

### TECHNICAL FIELD

The present invention relates to a nasal dilator which comprises a means for dilating the nostrils and a thermal element, which can be worn on the nose of a human for an extended period of time. Also disclosed is a method of treatment for relief of nasal blockage, sinus discomfort and pain, and other cold symptoms associated therewith, as well as related symptoms associated with allergies, by applying said nasal dilator to the nose of a human in need of such treatment.

### BACKGROUND OF THE INVENTION

Blockage of the nasal passages is obviously an inconvenience to persons who experience it. Blockage of the nasal passages is particularly uncomfortable at night, since it can lead to sleep disturbances, sleep irregularities, and/or snoring. In addition, a person with such a condition may wake often because he/she is not inhaling sufficient quantities of oxygen.

While there is a small portion of the human population which has some type of malformation of the nasal passages, such as a deviated septum, the majority of people who experience blockage of the nasal passages usually are suffering from the nasal congestion and other symptoms associated with the common cold. The common cold, although not usually a serious illness, is a highly prevalent, discomforting and annoying affliction. The term "common cold" is applied to minor respiratory illnesses caused by a variety of different respiratory viruses, of which rhinoviruses are the major known cause of common colds, accounting for approximately 30 percent of the colds in adults.

With the common cold, symptoms of nasal discharge, nasal congestion/blockage, and sneezing usually commence on the first day of illness and progress to maximum severity by the second or third day. Other symptoms may include mild burning of the eyes, loss of smell and taste, a feeling of pressure or fullness in the sinuses, sinus pain, headache, and vocal impairment. Many of these symptoms are shared by sufferers of allergies.

At present, treatment for the nasal congestion/blockage, sinus discomfort and pain, and other cold symptoms, including fever and the general malaise associated therewith, generally contain an analgesic (aspirin or acetaminophen) and one or more antihistamines. decongestants, cough suppressants, antitussives and expectorants; the majority of these drugs are taken orally. Other specific pharmaceutical actives for nasal symptoms (e.g., congestion) generally contain either oxymetazoline or phenylephrine and are generally delivered topically to the nasal mucosa via a nasal spray.

Nasal delivery of therapeutic agents has been well known for a number of years. See, for example, U.S. Patent 4,749,700 to Wenig, issued June 7, 1988, U.S. Patent 4,778,810 to Wenig, et al., issued October 18, 1988 and U.S. Patent 4,729,997 to Wenig issued March 8, 1988. Nasal saline sprays have been used to moisturize nasal passages and to dissolve build-up in the nasal mucosa; however, saline solutions alone have not proved satisfactory for relief of nasal congestion. Menthol has been administered orally from lozenges and the like as well as delivered to the nasal mucosa from an inhaler containing a wick, see for example, Clinical Otolaryngology, 1988, vol. 13, pp. 25-29. Yet menthol delivered in such a manner has not been found to provide a sufficient level of relief.

Another method of treating the nasal congestion/blockage, sinus pain, and other cold symptoms described above, is by application of heat to the nose and/or sinus areas. Such heat treatments include the use of hot towels and reusable thermal packs containing water and/or microwaveable gels. In general, such devices, which require the thermal source to be replenished, are inconvenient to use. Further, many of these thermal units or devices do not provide long lasting heat or maintain a consistent temperature over long periods of time. The beneficial therapeutic effects from this administration of heat diminishes after the heat source is removed; therefore, it is desirable to provide a sustained heat source to the afflicted area for as long as possible, preferably for about eight hours. These devices are also inconvenient to use at night. when the treatment is most often needed.

Nasal dilators for aiding breathing through the nose are known, however, these devices are also not generally effective in relieving nasal congestion/blockage, sinus discomfort and pain, and other cold/allergy symptoms. U.S. Patent No. 4,414,977, issued to Rezakhany, discloses one such nasal dilator. The nasal dilator includes generally elongated top and bottom rings which are spaced apart and connected together by a rear strut and a front strut. The front strut is longer than the rear strut and includes a bend therein formed at a position close to the front end of the bottom ring. When in place in the nasal passage, the top ring fits in the nasal valve within the nostril to prevent the tissue from being drawn in during inhalation, and to reduce extra flow resistance during exhalation. The bottom ring fits above the entrance to the nostril and serves to stabilize the position of the top ring within the nasal passage. One of these nasal dilators must be inserted into each nasal passage to provide unobstructed breathing. These nasal dilators. however, are not always effective since they are uncomfortable to wear, may cause irritation and itching of the nostril. unsafe to use at night during sleep, and are inconvenient to use when the wearer has nasal drainage due to a cold.

Another nasal dilator is disclosed in U.S. Patent No. 1,292,083, issued to Sawyer. This nasal dilator includes pads of adhesive material to which are attached metal loops. The pads are applied to the exterior surface of the nose above the nostrils. Once the pads are affixed, a dilating member is connected with each of the loops. The dilating member consists of a metal wire that provides a spring force which is directed outward or upward when hooked ends of the dilating member are engaged with the loops of the pads. A further nasal dilator is disclosed in U.S. Patent No. 1,950,839, issued to Chirila. This nasal dilator is similar to that of Sawyer but employs suction cups to secure a dilating member to the exterior surface of the nose. These dilators are not always effective because the dilating members can easily become disengaged from the pads or suction cups that secure the dilating members to the exterior of the nose. which could cause injury to the face or eyes, particularly during sleep.

Other nasal dilators are disclosed in U.S. Patent No. 5,533,499, issued to Johnson. U.S. Patent No. 5,533,503, issued to Doubek, et al., and U.S. Patent No. 5,546,929. issued to Muchin. These nasal dilators comprise a truss comprising a flexible strip and spring member which traverses the bridge of the nose. The flexible strip adheres to the exterior surface of the nose such that the ends of the truss member stabilize the outer wall of the nostrils, thereby preventing the outer wall from drawing in during breathing.

While the above described nasal dilators may aid breathing through the nose in a healthy person, it is evident that there is a continuing need for an improved means of treating the nasal congestion/blockage, sinus discomfort and pain, and other cold/allergy symptoms associated therewith. Specifically, there is a need for a nasal dilator that can provide safe and effective relief of these symptoms. Moreover, there is a need for a nasal dilator that can be reliably worn at night when the nasal congestion/blockage problem is most acute and most uncomfortable. In addition, there is a need for a nasal dilator that can be reliably worn through extended therapeutic periods without discomfort to the wearer. The nasal dilator should also be of efficient design and relatively uncomplicated.

The inventor of the present invention has developed a nasal dilator which comprises a means for dilating congested and/or blocked nasal passages due to the common cold and/or allergies and a thermal element to relieve the sinus discomfort and pain, and other cold/allergy symptoms associated therewith, which can be safely and comfortably worn, day or night, on the nose of a human for an extended period of time. The present inventor has also discovered a method of treatment for relief of the nasal congestion/blockage, sinus discomfort and pain. and other cold/allergy symptoms associated therewith, by applying said nasal dilator to the nose of a human in need of such treatment.

It is therefore an object of the present invention to provide a nasal dilator which comprises a means for dilating the nostrils and a thermal element which can be safely and comfortably worn, day or night, on the nose of a human for an extended period of time.

It is also an object to provide a method of treatment for relief of the nasal congestion/blockage, sinus discomfort and pain, and other cold symptoms associated therewith, by applying said nasal dilator to the nose of a human in need of such treatment.

It is a further object to provide a method of treatment for relief of the nasal congestion/blockage, sinus discomfort and pain, sneezing, and other symptoms associated with allergies, by applying said nasal dilator to the nose of a human in need of such treatment.

These objectives and additional objectives will become readily apparent from the detailed description which follows.

### SUMMARY OF THE INVENTION

The present invention comprises a nasal dilator which comprises a unitary truss member having an elongated shape and a normally, substantially planar state. The truss member further comprises a strip of flexible base material having a first side and a second side, a first end region adapted to engage the outer nasal tissue of a first nasal passage and a second end region adapted to engage the outer nasal tissue of a second nasal passage, coupled to one another by an intermediate segment configured to traverse the bridge of the nose of a human. The truss member is held in place on the nose of a human by a layer of an adhesive substance, which extends over the first and second end regions and intermediate segment of the first side of the flexible base material. The truss member acts to draw the outer nasal tissues of the first and second nasal passages outward, by way of a resilient means, comprising at least one resilient member which extends along the flexible base material and is oriented substantially parallel to a longitudinal extent of and fixably attached to the second side of the flexible base material. The truss member also comprises an thermal element comprising a thermogenic composition capable of providing heat preferably an exothermic composition comprising iron oxidation chemistry. The truss member still further comprises a strip of flexible top material having a first side and a second side. The peripheral edges of first side of the strip of flexible top material is bonded to the peripheral edges of the second side of the flexible base material to seal the resilient means and the thermogenic composition between the flexible top and base materials. At least one of the flexible top and base materials may be oxygen-permeable or made oxygen-permeable by penetrating one of the top and base materials with an array of pins, such that when the truss member is removed from its air-impermeable secondary package, the exothermic composition is activated and begins to generate controlled and sustained heat.

The nasal dilator may be used in a method of treatment to open the nasal passages blocked by congestion and/or swelling associated with the common cold and/or allergies and encourage free breathing. Such a treatment relieves the symptoms of nasal discharge, nasal congestion/blockage, and sneezing, as well as other symptoms which may include mild burning of the eyes, loss of smell and taste, a feeling of pressure or fullness in the sinuses, sinus pain, headache, and vocal impairment by applying said truss member to the nose of a human in need of such treatment.

### BRIEF DESCRIPTION OF THE DRAWINGS

FIG. 1 is a multiple perspective view of a portion of a face with a nasal dilator in accordance with a preferred embodiment of the present invention secured to a nose wherein:
   a. FIG. 1a is a perspective view of a portion of a face with a nasal dilator, as described in FIG. 2, secured to a nose;
   b. FIG. 1b is a perspective view of a portion of a face with a nasal dilator, as described in FIG. 3, secured to a nose; and
   c. FIG. 1c is a perspective view of a portion of a face with a nasal dilator, as described in FIG. 4, secured to a nose.
FIG. 2 is an exploded perspective view showing the components of one embodiment of the nasal dilator which comprises a single resilient member and wherein the intermediate segment of the truss is the same width as the width of the first and second ends of the truss.
FIG. 3 is an exploded perspective view showing the components of one embodiment of the nasal dilator which comprises two resilient members and wherein the intermediate segment of the truss has a width less than the width of the first and second ends of the truss.
FIG. 4 is an exploded perspective view showing the components of another embodiment of the nasal dilator described in FIG. wherein the components of the nasal dilator comprises two resilient members, two separate compartments of particulate exothermic composition, and the intermediate segment of the truss has a width less than the width of the first and second ends of the truss.
FIG. 5 is a multiple sectional view of a portion of a face showing the nose:
   a. FIG. 5a is a sectional view of a portion of a face showing a nose and nasal passages in a normal state of breathing;
   b. FIG. 5b is a sectional view of a portion of a face showing a nose and nasal passages in a state of swelling and/or congestion; and
   c. FIG. 5c is a sectional view of a portion of a face showing a nose and nasal passages with a nasal dilator, as described in FIGS. 1-4, secured to a nose.

### DETAILED DESCRIPTION OF THE INVENTION

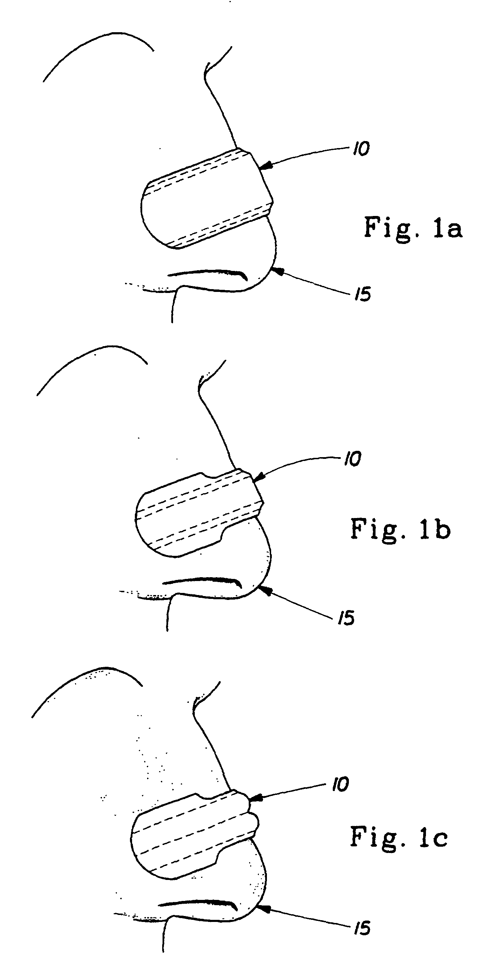

A nasal dilator 10 in accordance with a preferred embodiment of the present invention is illustrated generally in FIGS. 1a, 1b, and 1c. The nasal dilator 10 is shown secured to the nose 15 of a human.

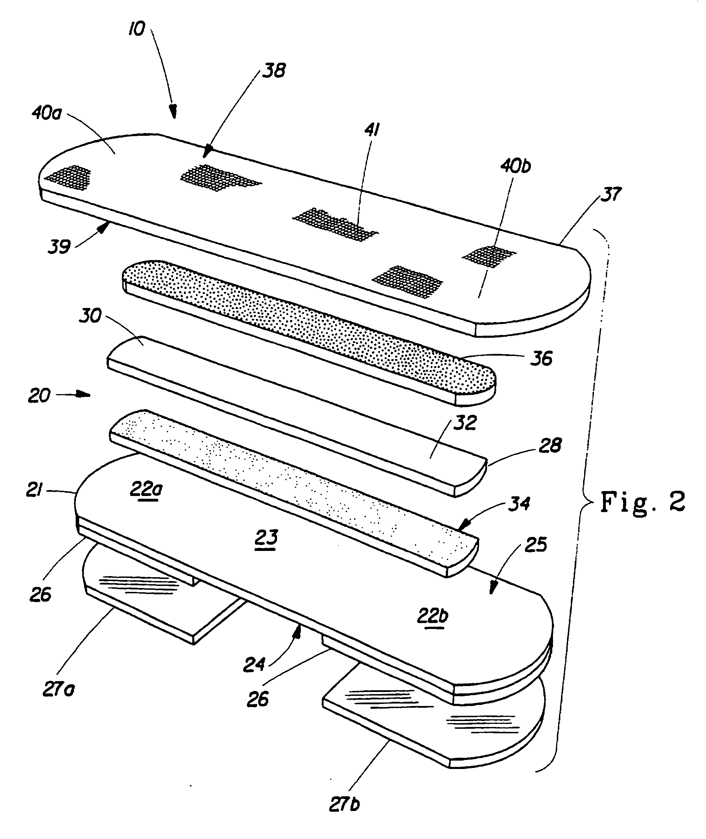

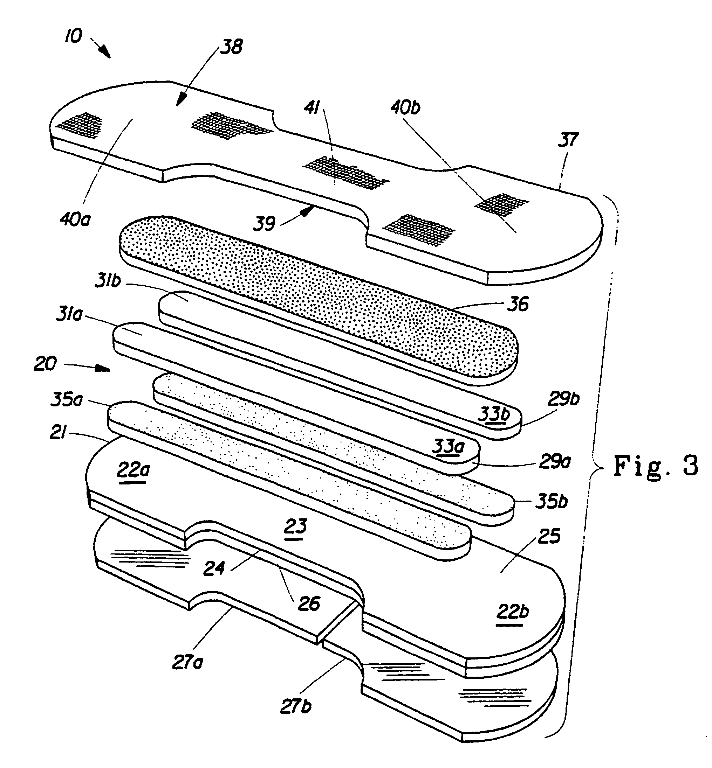

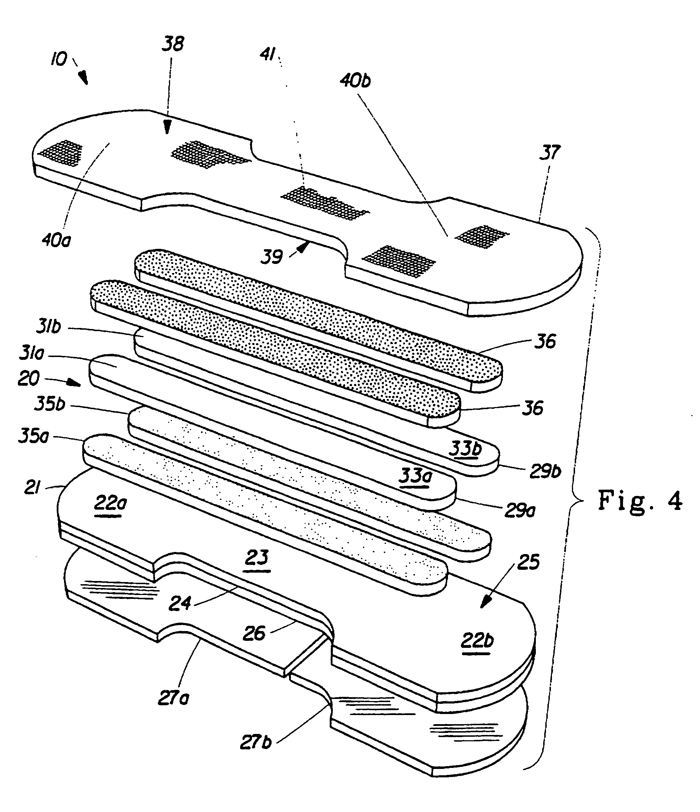

The nasal dilator 10, shown in FIG. 2, FIG. 3. and FIG. 4 comprises a unitary truss member 20, having an elongated shape and a normally, substantially planar state, and which includes a strip of flexible base material 21 having a first end region 22a, adapted to engage a first outer wall tissue 43 of a first nasal passage 45, and a second end region 22b, adapted to engage a second outer wall tissue 44 of a second nasal passage 46, coupled by an intermediate segment 23, configured to traverse the bridge of the nose 15 of a human. While the intermediate segment 23 may be the same width as the width of the first and second end regions 22a and 22b, as shown in FIG. 2, the preferred width of the intermediate segment 23 is less than the width of the first and second end regions 22a and 22b, as shown in FIGS. 3 and 4. The strip of flexible base material 21 also comprises a first side 24 and a second side 25. The strip of flexible base material 21 may be made of any suitable material. However, the preferred materials for the strip of flexible base material 21 are film layer substrates. The first side 24 of the strip of flexible base material 21 is typically made of nonwoven fabric, to provide support, laminated to the second side 25 of the strip of flexible base material 21 which is a film having heat sealability and capable of being easily thermally fused. The second side 25 may also be a liquefied silicone rubber coating applied to the non-woven fabric of the first side 24. For the non-woven fabrics of the first side 24, those having preferred characteristic properties of light weight and high tensile strength, e.g., nylon, rayon, cellulose ester, polyvinyl derivatives, polyolefins, polyamides, or polyesters, cuproammonium cellulose (Bemberg) and other high molecular weight compounds, as well as natural materials such as, wool, silk, jute, hemp, cotton, linen, sisal, or ramie, are suitable. These nonwoven materials are generally described in Riedel "Nonwoven Bonding Methods and Materials", Nonwoven World, (1987). Examples of the film of the second side 25 are polyethylene, polypropylene. nylon. polyester. polyvinyl chloride. polyvinylidene chloride. polyurethane, polystyrene. saponified ethylene-vinyl acetate copolymer. ethylene-vinyl acetate copolymer, natural rubber, reclaimed rubber and synthetic rubber. These materials may also be coextruded with low melt temperature polymers. The strip of flexible base material 21 thickness is in the range of about 1 to about 300 µm and may be oxygen permeable or impermeable. A preferred strip of flexible base material 21 of the present invention comprises a first side 24 of a polypropylene nonwoven sheet laminated to a second side 25 film of low-density polyethylene (LDPE), having a thickness of about 5 to about 100 µm.

Web material composed of continuous filaments of thermoplastic resin laminated with a thermoplastic resin film, such as those described in Japanese Kokai Patent Application No. HEI 07-067907, published March 14, 1995 may also be useful in the present invention.

The strip of flexible base material 21 also comprises on its first side 24. a layer of an adhesive substance 26 which extends over the first end region 22a and second end region 22b, preferably over the first end region 22a, second end region 22b, and the intermediate segment 23, of the strip of flexible base material 21. The adhesive substance 26 is preferably a breathable, acrylic, pressure sensitive, bio-compatible adhesive. Readily removable, first and second release liners 27a and 27b, respectively, cover the adhesive substance 26 on the first end region, second end region, and intermediate segment 22a, 22b, and 23 on the first side 24 of the strip of flexible base material 21. The first release liner 27a and second release liner 27b cover the adhesive substance 26 and remain in place on the strip of flexible base material 21 until the nasal dilator 10 is to be used.

The unitary truss member 20 further comprises a resilient means extending along said strip of flexible base material 21 such that said resilient means is oriented substantially parallel to a longitudinal extent of said strip of flexible base material 21 and secured to the second side 25 of the strip of flexible base material 21. The resilient means may comprise a single resilient member 28, as shown in FIG. 2, or a first resilient member 29a and a second resilient member 29b, as shown in FIGS. 3 and 4. The resilient members have a first end 30, 31a, and 31b, and a second end 32, 33a, and 33b, respectively. The resilient members 28, 29a, and 29b typically terminate at the end edges of the first and second end regions 22a and 22b of the strip of flexible base material 21. However, the resilient members 28, 29a, and 29b may terminate anywhere within the first and second end regions 22a and 22b of the strip of flexible base material 21. The resilient members 28, 29a, and 29b may be made from any suitable material having the appropriate axial and torsional flexibility, such as metal and/or plastic. The preferred material for the resilient members 28, 29a. and 29b is an industrial grade. biaxially oriented polyester that is approximately 2 mm to 8 mm wide and 0.25 mm thick. and may optionally have a plurality of grooves that extend substantially parallel to the respective resilient members 28, 29a, and 29b. The grooves create areas of reduced material to enhance the flexibility of the resilient members 28, 29a, and 29b in a direction perpendicular to the plurality of grooves.

The resilient members 28, 29a, and 29b are secured to the second side 25 of the flexible base material 21 by one or more strips of flexible adhesive material 34, 35a, and 35b. The strips of flexible adhesive material 34, 35a, and 35b are of the same amount. size, and shape as the resilient members 28, 29a, and 29b, respectively. Each strip of flexible adhesive material 34, 35a, and 35b is preferably a double-sided adhesive, foam tape or an acrylic, pressure sensitive bio-compatible adhesive material, such as 3M-1509, available from Minnesota, Mining, & Manufacturing, Inc., St. Paul, MN.

The unitary truss member 20 further comprises a strip of flexible top material 37 having a first side 38, a second side 39, a first end region 40a, a second end region 40b, and an intermediate segment 41. The first end region 40a, second end region 40b, and intermediate segment 41 are of the same size and shape as the first end region 22a, second end region 22b, and intermediate segment 23, respectively, of the strip of flexible base material 21. The strip of flexible top material 37 may be made of any suitable material. However, the preferred materials for the strip of flexible top material 37 is generally the same as for the strip of flexible base material 21, that is, film layer substrates wherein the first side 38 is a nonwoven fabric, to provide support, laminated to the second side 39 which is a film having heat sealability and capable of being easily thermally fused. The strip of flexible top material 37 thickness is in the range of about 1 to about 300 µm and may be oxygen permeable or impermeable. The preferred film layer substrates for the strip of flexible top material 37 comprise a first side 38 of polypropylene nonwoven sheets laminated to a second side 39 of a film of poly(ethylene-vinyl acetate), having a thickness of about 5 to about 100 µm.

The unitary truss member 20 further comprises a thermal element. Most any thermal composition, such as exothermic compositions, microwaveable compositions, heat of crystallization compositions, and the like, as well as compositions capable of producing a cooling effect, may be used. However, the thermal element of the present invention is thermogenic and comprises a particulate exothermic composition 36. While the particulate exothermic composition 36 may comprise any composition capable of generating heat, the particulate exothermic composition 36 preferably comprises, but is not limited to, powdered iron, powdered carbon, metal salt, and water. Compositions of this type react when exposed to oxygen providing heat for several hours. In the alternative, the unitary truss member 20 may comprises a composition capable of producing a cooling effect.

The particulate exothermic composition 36 typically comprises from about 30% to about 80% iron powder, from about 3% to about 25% activated carbon, non-activated carbon, and mixtures thereof. from about 0.5% to about 10% of a metal salt, and from about 1% to about 40% water.

Suitable sources for iron powder include cast iron powder, reduced iron powder. electrolytic iron powder, scrap iron powder, pig iron, wrought iron, various steels, iron alloys, and the like and treated varieties of these iron powders. There is no particular limitation to their purity, kind, etc. so long as it can be used to produce heat-generation with electrically conducting water and air.

Active carbon prepared from coconut shell, wood, charcoal, coal, bone coal, etc. is useful, but those prepared from other raw materials such as animal products, natural gas, fats, oils and resins are also useful in the present invention. There is no limitation to the kinds of active carbon used, however, the preferred active carbon has superior water holding capabilities. The capabilities of the carbon can be extended by using mixtures of the above carbons, i.e., active and non-activated carbon powders blended to reduce cost. Therefore, mixtures of the above carbons are useful in the present invention as well.

Useful metal salts include sulfates such as ferric sulfate, potassium sulfate, sodium sulfate, manganese sulfate, magnesium sulfate; and chlorides such as cupric chloride, potassium chloride, sodium chloride, calcium chloride, manganese chloride, magnesium chloride and cuprous chloride. Also, carbonate salts, acetate salts, nitrates. nitrites and other salts can be used. Among these metal salts, the deliquescent salts such as calcium chloride, magnesium chloride, etc. are very hygroscopic and hence these compounds, even when added in a small amount, show an effectiveness in inhibiting the escape of water vapor. Sodium chloride shows small solubility difference vs. temperature difference and hence no crystal is precipitated at low temperatures, and also provides reasonable heat-generation. Thus, deviation of heat-generation due to temperature difference of atmospheric air does not occur. In general, several suitable alkali, alkaline earth, and transition metal salts exist which can also be used, alone or in combination, to sustain the corrosive reaction of iron. The preferred metal salts of the present invention are sodium chloride, cupric chloride, and mixtures thereof.

The water used herein may be from any appropriate source. There is no particular limitation to its purity, kind, etc.

While oxygen is necessary for the oxidation reaction of iron to occur, an internal oxygen source is not required in the present invention, however, oxygen-producing chemical materials may be incorporated in the particulate exothermic composition at the time of preparation thereof without changing the scope of the present invention. The oxygen sources used for the purpose of this invention include air and artificially made oxygen of various purity. Among these oxygen sources. air is preferred since it is the most convenient and inexpensive.

In addition to the above described components of the particulate exothermic compositions of the present invention, other components may also be added as appropriate, such as additional water-holding materials including vermiculite. porous silicates, wood powder, wood flour, cotton cloth having a large amount of fluffs, short fibers of cotton, paper scrap, vegetable matter, super absorbent water-swellable or water-soluble polymers and resins, carboxymethylcellulose salts, and other porous materials having a large capillary function and hydrophilic properties; agglomeration aids including gelatin, natural gums, cellulose derivatives, cellulose ethers and their derivatives, starch, modified starches, polyvinyl alcohols, polyvinylpyrrolidone, sodium alginates, polyols, glycols, corn syrup, sucrose syrup, sorbitol syrup and other polysaccharides and their derivatives, polyacrylamides, polyvinyloxoazolidone, and maltitol syrup; dry binders including maltodextrin, sprayed lactose, co-crystallized sucrose and dextrin, modified dextrose, sorbitol, mannitol, microcrystalline cellulose, microfine cellulose, pre-gelatinized starch, dicalcium phosphate, and calcium carbonate; oxidation reaction enhancers including elemental chromium, manganese, or copper, compounds comprising said elements, or mixtures thereof; hydrogen gas inhibitors including inorganic or organic alkali compounds or alkali weak acid salts such as sodium hydroxide, potassium hydroxide, sodium hydrogen carbonate, sodium carbonate, calcium hydroxide, calcium carbonate, and sodium propionate; fillers including natural cellulosic fragments including wood dust, cotton linter, and cellulose, synthetic fibers in fragmentary form including polyester fibers, foamed synthetic resins such as foamed polystyrene and polyurethane, and inorganic compounds including silica powder, porous silica gel, sodium sulfate, barium sulfate, iron oxides, and alumina; and anti-caking agents including tricalcium phosphate and sodium silicoaluminate. Such components also include thickeners such as cornstarch, potato starch, carboxymethylcellulose, and α-starch, and surfactants such as those included within the anionic, cationic, nonionic, zwitterionic, and amphoteric types. Still other additional components which may be added to the particulate exothermic compositions of the present invention, as appropriate, include extending agents such as metasilicates, zirconium, and ceramics.

Preferably at least 50%, more preferably 70%, even more preferably 80% and most preferably 90% of all of the particles by weight of the particulate exothermic composition of the present invention have a mean particle size of less than 200 µm. preferably less than 150 µm.

The above-mentioned components of the composition may be blended while being isolated from air using conventional blending techniques. Suitable methods of blending these components are described in detail in U. S. Patent 4,649.895 to Yasuki et al., issued March 17, 1987. For example, carbon is added to a blender or mixer, followed by water and this combination is mixed. Usually enough water is added to assist in blending while avoiding escalated corrosion. e.g., 3.5% by weight of the particulate composition. Mixing is stopped and. in the absence of air, vermiculite and sodium chloride are added together. Mixing is resumed until all the components are mixed thoroughly and iron powder is added and mixed. The composition is then blended until thoroughly mixed. Additional water is added to the particulate composition during construction of the nasal dilator of the present invention. The above method may be modified as required, such as the salt and additional water may be added to the particulate composition as brine during construction of the nasal dilator.

In the alternative, the dry powdered components of the present invention, except water, may be blended, using conventional blending techniques and agglomerated into granules. For example, powdered carbon and a metal salt are added to a blender or mixer, and blended into a uniform dry mixture. An additional water-holding material is added and the composition is mixed until uniform. For this particular method, dry binders may be optionally added to the composition along with the additional water-holding material. Powdered iron is added and the mixture is again blended until uniform. An agglomeration aid is then added to the blended powders. The composition is mixed until a light agglomeration is formed and no dust appears. The agglomerated granules useful in the exothermic compositions of the present invention are easily wetted, less dense particles and soft porous granules. The granules formed by the agglomeration process may be optionally "rounded" on a rotary granulator, and fines reattached prior to being placed into a nasal dilator of the present invention. While the above described method of making the exothermic composition is by dry agglomeration, wet agglomeration techniques may also be used.

Individual nasal dilators 10 can typically be prepared by bonding the strip of flexible base material 21 to the strip of flexible top material 37 around their peripheral edges, such that the second side 25 of the strip of flexible base material 21 faces and is fused to the second side 39 of the strip of flexible top material 37. This forms a pouch, envelope, or pocket with the second side 25 of the strip of flexible base material 21 and the second side 39 of the strip of flexible top material 37 toward the inside of the pouch. envelope, or pocket and the first side 24 of the strip of flexible base material 21 and the first side 38 of the strip of flexible top material 37 toward the outside, thereby sealing the resilient members 28, 29a, and 29b, strips of flexible adhesive material 34, 35a, and 35b, and particulate exothermic composition 36. inside the pouch, envelope, or pocket, and thereby forming a unified structure which forms the nasal dilator 10 of the present invention. Bonding of the strip of flexible base material 21 to the strip of flexible top material 37 around their peripheral edges is typically done using a low heat, however, other means, such as an adhesive, may also be used.

Oxygen permeability can be provided by selecting films or film coatings for the second side 25 of the strip of flexible base material 21 and the second side 39 of the strip of flexible top material 37 forming the pouches, envelopes, pockets, that have the specifically desired permeability properties. Oxygen permeability can also be provided in the present invention by perforating the strip of flexible top material 37 with aeration holes using, for example, at least one pin, preferably an array of from about 20 to about 60 pins, with, e.g., tapered points and diameters of from about 0.2 mm to about 2 mm. preferably from about 0.4 mm to about 0.9 mm. The pins are pressed through the first and second side 38 and 39 of the strip of flexible top material 37 to a depth of from about 2% to about 100%, preferably from about 20% to about 100%, and more preferably from about 50% to about 100% into the particulate exothermic composition 36. This hole configuration provides an oxygen diffusion into the particulate exothermic composition 36 during the oxidation reaction of from about 0.01 cc O₂/min./5 cm² to about 15.0 cc O₂/min./5 cm² (at 21°C, 1 ATM), preferably from about 0.9 cc O₂/min./5 cm² to about 1.1 cc O₂/min./5 cm² (at 21 °C, 1 ATM). Although there is preferably provided aeration holes in the strip of flexible top material 37, it is also possible to provide aeration holes in the strip of flexible base material 21, and/or both.

The velocity, duration, and temperature of the thermogenic oxidation reaction of the particulate exothermic composition can be controlled as desired by changing the area of contact with air, more specifically, by changing the oxygen diffusion/permeability.

The nasal dilator 10 may optionally incorporate a component, added as a separate substrate layer between the strip of flexible base material 21 and the layer of adhesive substance 26, incorporated into the strip of flexible top material 37 and/or the strip of flexible base material 21, or added to the particulate exothermic composition 36, comprising active aromatic compounds, non-active aromatic compounds, pharmaceutical actives or other therapeutic agents, and mixtures thereof, to be delivered through the skin. Such active aromatic compounds include, but are not limited to, menthol. camphor, eucalyptus. and mixtures thereof. Such non-active aromatic compounds include, but are not limited to, benzaldehyde. citral, decanal. aldehyde, and mixtures thereof. Such pharmaceutical actives/therapeutic agents include, but are not limited to, decongestants, antitussive agents, antihistamines. antibiotics. vitamins, antiviral agents, analgesics, anti-inflammatory agents including non-steriodal anti-inflammatory agents, antipruritics, antipyretics, anesthetic agents, antifungals, antimicrobials, and mixtures thereof.

The finished nasal dilator 10 is packaged, by enclosing the nasal dilator 10, in a secondary air-impermeable package to prevent the oxidation reaction from occurring until desired as described in the aforementioned U.S. Patent 4,649,895. The nasal dilator remains sealed inside the air-impermeable package until a user is ready to apply the nasal dilator 10 to said user's nose 15, whereby opening the air-impermeable package enables oxygen from ambient air to activate the heating element to generate controlled and sustained heating. Alternatively, air impermeable removable adhesive strips can be placed over the aeration holes in the strip of flexible top material 37 such that, when the strips are removed, air is allowed to enter the strip of flexible top material 37, thus activating the oxidation reaction of the iron powder.

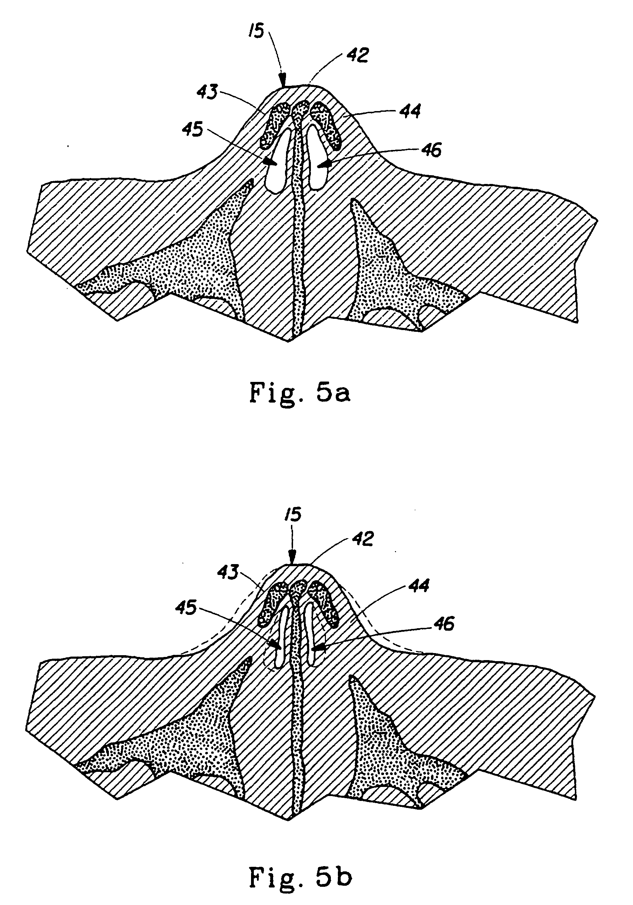

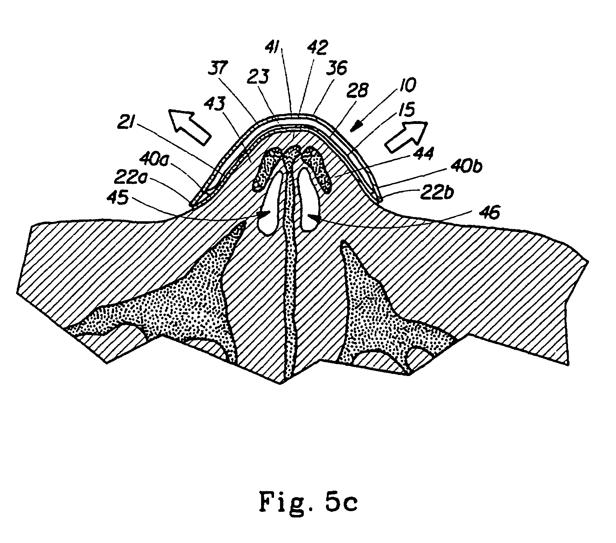

To secure the nasal dilator 10 to the nose 15, the first and second release liners 27a and 27b are removed from the strip of flexible base material 21 to expose the adhesive substance 26. As seen in FIGS. 1 and 5, the nasal dilator 10 is placed on the exterior of the nose 15 such that the nasal dilator 10 traverses the bridge of the nose 42 and the first and second end regions 22a and 22b of the strip of flexible base material 21 contact the first and second outer wall tissue 43 and 44 of the first and second nasal passages 45 and 46 of the nose 15. The adhesive substance 26 on the first and second end regions 22a and 22b and the intermediate segment 23 of the strip of flexible base material 21 releasably secures the unitary truss member 20 to the bridge of the nose 42 and to the first and second outer wall tissue 43 and 44 of the first and second nasal passages 45 and 46 of the nose 15.

With the nasal dilator 10 in place about the nose 15, the resiliency of the resilient members 28, 29a, and 29b act to stabilize the outer wall tissue 43 and 44 of the nose 15 and thereby draws the outer wall tissue 43 and 44 of the nose 15 outward. Moreover, the flexibility of the base material 21, strips of flexible adhesive material 34, 35a, and 35b and top material 37, the resiliency and flexibility of the resilient members 28, 29a, and 29b, all allow the nasal dilator 10 of the present invention to closely conform to the curves of the nose 15 of each individual wearer. The relative slight thickness of the material of the resilient members 28, 29a, and 29b, also enhances axial, torsional flexibility of the truss member 20 about the longitudinal extent of the truss member 20. which increases wearer comfort and facilitates adhesion of the adhesive substance 26.

The desired functional range of dilating force (i.e., the spring biasing force due to the resiliency of the resilient members 28, 29a, and 29b, of the nasal dilator 10) is typically in the range of from about 5 grams to about 50 grams. Therefore, the nasal dilator 10 of the present invention is constructed to provide from about 5 grams to about 50 grams. preferably from about 10 grams to about 40 grams. and more preferably from about 20 to about 30 grams of dilating spring biasing force to each outer wall tissue 43 and 44 of the nasal passage 45 and 46 of the nose 15.

The nasal dilator 10 is an efficient design that can be efficiently manufactured. Moreover, this nasal dilator 10 can be worn reliably at night when the inhalation nasal blockage problem is most acute, without anxiety and inconvenience normally associated with other nasal dilators. In addition. the nasal dilator 10 can be comfortably worn through extended therapeutic periods.

Although the present invention has been described with reference to the preferred embodiments, those skilled in the an will recognize that changes may be made in form and detail. such as those described in U.S. Patent No. 5,533,499, issued to Johnson. U.S. Patent No. 5,533,503, issued to Doubek, et al., and U.S. Patent No. 5,546,929, issued to Muchin.

The nasal dilator 10 may be applied to the nose 15 of a person suffering from symptoms of nasal discharge. nasal congestion/blockage, and sneezing, as well as other symptoms which may include mild burning of the eyes, loss of smell and taste, a feeling of pressure or fullness in the sinuses, sinus pain, headache, and vocal impairment usually associated with the common cold and/or allergies, for comfortable and convenient relief of said symptoms for an extended period of time, i.e.. at least 8 hours.

## Claims

1. A nasal dilator (10) comprising a unitary truss member (20) having an elongated shape and a planar state when unapplied, comprising:
a. a strip of flexible base material (21) having a first side (24) and a second side (25); a first end region (22a) adapted to engage the outer wall tissue (43) of a first nasal passage (45); a second end region (22b) adapted to engage the outer wall tissue (44) of a second nasal passage (46); an intermediate segment (23) coupling said first end region (22a) to said second end region (22b) and configured to traverse a portion of a nose (15) located between said first nasal passage (45) and said second nasal passage (46); and a layer of an adhesive substance (26) extending over said first end region (22a) and said second end region (22b) of said first side (24) of said strip of flexible base material (21);
b. a resilient means (28, 29a, 29b) fixably attached to said second side (25) of said strip of flexible base material (21) and extending along said strip of flexible base material such that said resilient means is oriented parallel to a longitudinal extent of said strip of flexible base material, and
c. a strip of flexible top material (37) having a first side (38) and a second side (39), wherein said second side of said strip of flexible top material is fixably attached around its peripheral edges to the peripheral edges of said second side of said strip of flexible base material such that said resilient means and said heating element are sealed between said strip of flexible base material and said strip of flexible top material;
wherein the inherent tendency of said unitary truss member (20) is to return to a planar state when flexed to engage said outer wall tissue (43, 44) of said first nasal passage (45) and second nasal passage (46) so as to pull said outer wall tissue outward, **characterised in that** said nasal dilator (10) comprises a thermal element comprising a thermogenic composition (36) capable of providing heat.

2. The nasal dilator (10) of claim 1 wherein the first side (24) of the strip of flexible base material (21) comprises a non-woven fabric laminated to the second side (25) which comprises a film having heat sealability and is capable of being thermally fused.

3. The nasal dilator (10) of claim 1 wherein the first side (38) of the strip of flexible top material (37) comprises a non-woven fabric laminated to the second side (39) which comprises a film having heat sealability and is capable of being thermally. fused.

4. A nasal dilator (10) according to claim 1 or claim 2 wherein the resilient means comprises at least one resilient member (28) having a first end (30) which terminates at end edge of said first end region (22a) of said strip of flexible base material (21) and a second end (32) which terminates at end edge of said second end region (22b) of said strip of flexible base material.

5. A nasal dilator (10) according to claim 3 wherein the at least one resilient member (28) is secured to said first end region (22a), said second end region (22b), and said intermediate segment (23) of said second side of said strip of flexible base material (21) by at least one strip of flexible adhesive material (34) having the same size and shape as said resilient member.

6. A nasal dilator (10) according to claim 1 wherein said thermal element (36) comprises particulate exothermic composition comprising:
a) from 30% to 80% iron powder;
b) from 3% to 25% carbonaceous material consisting of activated carbon, non-activated carbon, or mixtures thereof;
c) from 0.5% to 10% metal salt; and
d) from 1% to 40% water;
preferably said exothermic composition further comprises additional water-holding materials, dry binders, agglomeration aids, or mixtures thereof, more preferably wherein said exothermic composition is agglomerated granules.

7. A nasal dilator (10) according to claim 1 wherein said second side (25) of said strip of flexible based material (21) and said second side (39) of said strip of flexible top material (37) are formed from an oxygen-permeable film and are made oxygen-permeable by penetrating at least one of said strip of flexible base material (21) and said strip of flexible top material (37) with at least one pin, preferably from 20 to 60 pins, to form at least one aeration hole, preferably a plurality of aeration holes, having a diameter of from 0.2mm to 2mm, preferably from 0.4mm to 0.9mm.

8. A nasal dilator (10) according to any one of the preceding claims further comprising first (27a) and second (27b) release liners covering the adhesive substance (26) on said first end region (22a) and said second end region (22b) of said first side (24) of said strip of flexible base material (21), preferably covering the adhesive substance (26) on said first end region (22a), said second end region (22b) and intermediate segment (23) of said first side (24) of said strip of flexible base material (21), wherein said first and second release liners are readily removable from said strip of flexible base material (21) to expose said adhesive substance (26) and permit said unitary truss member (20) to be secured to said outer wall tissue (43, 44) of said first nasal passage (45) and said second nasal passage (46).

9. A nasal dilator (10) according to any one of the preceding claims wherein said first side (24) of said strip of flexible base material (21) comprises a pharmaceutical active or therapeutic agent to be delivered through the skin of the nose, preferably wherein a separate substrate layer comprising a pharmaceutical active or therapeutic agent to be delivered through the skin of the nose is added between said strip of flexible base material (21) and said layer of adhesive substance (26), more preferably wherein said pharmaceutical active or therapeutic agent comprises active aromatic compounds, non-active aromatic compounds, decongestants, antitussive agents, antihistamines, antibiotics, vitamins, antiviral agents, analgesics, anti-inflammatory agents including non-steroidal anti-inflammatory agents, antipruritics, antipyretics, anaesthetic agents, antifungals, antimicrobials, or mixtures thereof.

10. A nasal dilator (10) according to any one of the preceding claims further comprising an air-impermeable package enclosing said nasal dilator, wherein said nasal dilator remains sealed inside said air-impermeable package until a user is ready to apply said nasal dilator to said user's nose, whereby opening said air-impermeable package enables oxygen from ambient air to activate said heating element to generate controlled and sustained heating.

## Patentansprüche

1. Nasaldilatator (10), umfassend ein unitäres Verstärkungselement (20) mit einer länglichen Form und einem planaren Zusand, wenn es nicht aufgetragen ist, umfassend:
a. einen Streifen aus einem flexiblen Basismaterial (21) mit einer ersten Seite (24) und einer zweiten Seite (25); einen ersten Endbereich (22a), angepasst zum Erfassen des Außenwandgewebes (43) einer ersten Nasenpassage (45); einen zweiten Endbereich (22b), angepasst zum Erfassen des Außenwandgewebes (44) einer zweiten Nasenpassage (46); ein Zwischensegment (23), welches den ersten Endbereich (22a) an den zweiten Endbereich (22b) koppelt und derartig konfiguriert ist, dass es einen Teil einer Nase (15), welcher sich zwischen der ersten Nasenpassage (45) und der zweiten Nasenpassage (46) befindet, überquert; und eine Schicht einer adhäsiven Substanz (26), welche sich über den ersten Endbereich (22a) und den zweiten Endbereich (22b) der ersten Seite (24) des Streifens aus dem flexiblen Basismaterial (21) erstreckt;
b. ein elastisches Mittel (28, 29a, 29b), welches an der zweiten Seite (25) des Streifens aus dem flexiblen Basismaterial (21) fest angebracht ist und sich entlang des Streifens aus dem flexiblen Basismaterial derartig erstreckt, dass das elastische Mittel parallel zu einem Längsabschnitt des Streifens aus dem flexiblen Basismaterial orientiert ist, und
c. einen Streifen aus einem flexiblen Obermaterial (37) mit einer ersten Seite (38) und einer zweiten Seite (39), worin die zweite Seite des Streifens aus dem flexiblen Obermaterial entlang seiner peripheren Kanten an die peripheren Kanten der zweiten Seite des Streifens aus dem flexiblen Basismaterial derartig fest angebracht ist, dass das elastische Mittel und das Erwärmungselement zwischen dem Streifen aus dem flexiblen Basismaterial und dem Streifen aus dem flexiblen Obermaterial versiegelt sind;
worin die inhärente Tendenz des unitiären Verstärkungselements (20) zu einer Rückkehr zu einem planaren Zustand besteht, wenn es zum Erfassen des Außenwandgewebes (43, 44) der ersten Nasenpassage (45) und der zweiten Nasenpassage (46) gebogen ist, so dass das Außenwandgewebe nach außen gezogen wird, **dadurch gekennzeichnet, dass** der Nasaldilatator (10) ein thermisches Element umfasst, umfassend eine thermogene Zusammensetzung (36), welche zum Bereitstellen von Wärme in der Lage ist.

2. Nasaldilatator (10) nach Anspruch 1, worin die erste Seite (24) des Streifens aus dem flexiblen Basismaterial (21) ein Non-Woven-Gewebe, laminiert auf die zweite Seite (25), umfasst, welches eine Folie mit einer Wärmeversiegelbarkeit umfasst und zum thermischen Verschmelzen in der Lage ist.

3. Nasaldilatator (10) nach Anspruch 1, worin die erste Seite (38) des Streifens aus dem flexiblen Obermaterial (37) ein Non-Woven-Gewebe, laminiert auf die zweite Seite (39), umfasst, welches eine Folie mit einer Wärmeversiegelbarkeit umfasst und zum thermischen Verschmelzen in der Lage ist.

4. Nasaldilatator (10) nach Anspruch 1 oder 2, worin das elastische Mittel mindestens ein elastisches Element (28) mit einem ersten Ende (30), welches an der Endkante des ersten Endbereichs (22a) des Streifens aus dem flexiblen Basismaterial (21) endet, und einem zweiten Ende (32), welches an der Endkante des zweiten Endbereichs (22b) des Streifens aus dem flexiblen Basismaterial endet, umfasst.

5. Nasaldilatator (10) nach Anspruch 3, worin mindestens ein elastisches Element (28) an dem ersten Endbereich (22a), dem zweiten Endbereich (22b) und dem Zwischensegment (23) der zweiten Seite des Streifens aus dem flexiblen Basismaterial (21) durch mindestens einen Streifen aus einem flexiblen adhäsiven Material (34) mit der selben Größe und Form wie das elastische Element befestigt ist.

6. Nasaldilatator (10) nach Anspruch 1, worin das thermische Element (36) eine teilchenförmige exotherme Zusammensetzung umfasst, umfassend:
a) 30% bis 80% Eisenpulver;
b) 3% bis 25% Kohlenmaterial, bestehend aus Aktivkohle, Nichtaktivkohle, oder Mischungen davon;
c) 0,5% bis 10% Metallsalz; und
d) 1% bis 40% Wasser;
wobei die exotherme Zusammensetzung bevorzugt weiterhin zusätzlich wasserzurückhaltende Materialien, Trockenbindemittel, Agglomerationshilfsstoffe, oder Mischungen davon umfasst, besonders bevorzugt, worin die exotherme Zusammensetzung agglomerierte Granulate darstellt.

7. Nasaldilatator (10) nach Anspruch 1, worin die zweite Seite (25) des Streifens aus dem flexiblen Basismaterial (21) und die zweite Seite (39) des Streifens aus dem flexiblen Obermaterial (37) aus einer sauerstoffpermeablen Folie gebildet sind und durch Penetration mindestens eines der Streifen aus dem flexiblen Basismaterial (21) und dem Streifen aus dem flexiblen Obermaterial (37) durch mindestens einem Stift, bevorzugt 20 bis 60 Stiften, unter Bildung von mindestens einem Belüftungsloch, bevorzugt von mindestens einer Vielzahl von Belüftungslöchern, mit einem Durchmesser von 0,2 mm bis 2 mm, bevorzugt von 0,4 mm bis 0,9 mm, sauerstoffpermeabel gemacht sind.

8. Nasaldilatator (10) nach mindestens einem der vorangehenden Ansprüche, welcher weiterhin erste (27a) und zweite (27b) Abziehliner umfasst, welche die adhäsive Substanz (26) des ersten Endbereichs (22a) und des zweiten Endbereichs (22b) der ersten Seite (24) des Streifens aus dem flexiblen Basismaterial (21) bedecken, wobei sie bevorzugt die adhäsive Substanz (26) auf dem ersten Endbereich (22a), dem zweiten Endbereich (22b) und dem Zwischensegment (23) der ersten Seite (24) des Streifens aus dem flexiblen Basismaterial (21) bedecken, worin die ersten und zweiten Abziehliner von dem Streifen aus dem flexiblen Basismaterial (21) unter Freisetzung der adhäsiven Substanz (26) leicht entfernbar sind und es dem unitären Verstärkungselement (20) erlauben, an das Außenwandgewebe (43, 44) der ersten Nasenpassage (45) und der zweiten Nasenpassage (46) gesichert zu sein.

9. Nasaldilatator (10) nach mindestens einem der vorangehenden Ansprüche, worin die erste Seite (24) des Streifens aus dem flexiblen Basismaterial (21) einen pharmazeutischen Wirkstoff oder ein therapeutisches Mittel umfasst, welches durch die Haut der Nase verabreicht wird, bevorzugt worin eine separate Substratschicht, umfassend einen durch die Haut der Nase zu verabreichenden pharmazeutischen Wirkstoff oder therapeutisches Mittel, zwischen dem Streifen des flexiblen Basismaterials (21) und der Schicht aus der adhäsiven Substanz (26) eingesetzt ist, besonders bevorzugt, worin der pharmazeutische Wirkstoff oder das therapeutische Mittel aktive aromatische Verbindungen, nichtaktive aromatische Verbindungen, abschwellende Mittel, Antitüssiva, Antihistaminika, Antibiotika, Vitamine, antivirale Mittel, Analgetika, entzündungshemmende Mittel, einschließlich nichtsteroidialer entzündungshemmender Mittel, Antipruritika, fiebersenkende Mittel, Anästhetika, Antipilzmittel, antimikrobielle Mittel, oder Mischungen davon umfassen.

10. Nasaldilatator (10) nach mindestens einem der vorangehenden Ansprüche, welcher weiterhin eine luftundurchlässige Verpackung umfasst, welche den Nasaldilatator einschließt, worin der Nasaldilatator im Inneren der luftundurchlässigen Verpackung so lange bleibt, bis ein Verbraucher den Nasaldilatator auf die Nase eines Verbrauchers aufträgt, wobei das Öffnen der luftundurchlässigen Verpackung die Aktivierung des Erwärmungselements unter Bildung von regulierter beziehungsweise kontrollierter und anhaltender Wärme durch den Sauerstoff der Umgebungsluft ermöglicht.

## Revendications

1. Dilatateur nasal (10) comportant un élément de bandage unitaire (20) ayant une forme allongée et un état plan lorsqu'il n'est pas appliqué, comportant :
a. une bande de matériau de base flexible (21) ayant un premier côté (24) et un second côté (25), une première région d'extrémité (22a) adaptée pour venir en prise avec le tissu de paroi extérieure (43) d'un premier passage nasal (45), une seconde région d'extrémité (22b) adaptée pour venir en prise avec le tissu de paroi extérieure (44) d'un second passage nasal (46), un segment intermédiaire (23) reliant ladite première région d'extrémité (22a) à ladite seconde région d'extrémité (22b) et configuré pour traverser une partie d'un nez (15) située entre ledit premier passage nasal (45) et ledit second passage nasal (46), et une couche d'une substance adhésive (26) s'étendant sur ladite première région d'extrémité (22a) et ladite seconde région d'extrémité (22b) dudit premier côté (24) de ladite bande de matériau de base flexible (21),
b. des moyens élastiques (28, 29a, 29b) attachés de manière fixe audit second côté (25) de ladite bande de matériau de base flexible (21) et s'étendant le long de ladite bande de matériau de base flexible de sorte que lesdits moyens élastiques sont orientés parallèlement à une étendue longitudinale de ladite bande de matériau de base flexible, et
c. une bande de matériau supérieur flexible (37) ayant un premier côté (3 8) et un second côté (39), ledit second côté de ladite bande de matériau supérieur flexible étant attaché de manière fixe autour de ses rebords périphériques aux rebords périphériques dudit second côté de ladite bande de matériau de base flexible de sorte que lesdits moyens élastiques et ledit élément chauffant sont rendus étanches entre ladite bande de matériau de base flexible et ladite bande de matériau supérieur flexible,
dans lequel la tendance inhérente dudit élément de bandage unitaire (20) est de revenir dans un état plan lorsqu'il est fléchi pour venir en prise avec ledit tissu de paroi extérieure (43, 44) dudit premier passage nasal (45) et dudit second passage nasal (46) de manière à pousser ledit tissu de paroi extérieure vers l'extérieur, **caractérisé en ce que** ledit dilatateur nasal (10) comporte un élément thermique comportant une composition thermogénique (36) capable de fournir de la chaleur.

2. Dilatateur nasal (10) selon la revendication 1, dans lequel le premier côté (24) de la bande de matériau de base flexible (21) comporte un tissu non-tissé stratifié sur le second côté (25) qui comporte un film ayant une capacité d'étanchéité à la chaleur et est capable d'être fondu thermiquement.

3. Dilatateur nasal (10) selon la revendication 1, dans lequel le premier côté (38) de la bande de matériau supérieur flexible (37) comporte un tissu non-tissé stratifié sur le second côté (39) qui comporte un film ayant une capacité d'étanchéité à la chaleur et est capable d'être fondu thermiquement.

4. Dilatateur nasal (10) selon la revendication 1 ou la revendication 2, dans lequel les moyens élastiques comportent au moins un élément élastique (28) ayant une première extrémité (30) qui se termine au niveau d'un rebord d'extrémité de ladite première région d'extrémité (22a) de ladite bande de matériau de base flexible (2 1 ) et une seconde extrémité (32) qui se termine au niveau d'un rebord d'extrémité de ladite seconde région d'extrémité (22b) de ladite bande de matériau de base flexible.

5. Dilatateur nasal (10) selon la revendication 3, dans lequel au moins un élément élastique (28) est fixé à ladite première région d'extrémité (22a), à ladite seconde région d'extrémité (22b), et audit segment intermédiaire (23) dudit second côté de ladite bande de matériau de base flexible (21) par au moins une bande de matériau adhésif flexible (34) ayant les mêmes taille et forme que ledit élément élastique.

6. Dilatateur nasal (10) selon la revendication 1, dans lequel ledit élément thermique (36) comporte une composition exothermique particulaire, comportant :
a) de 30 % à 80 % de poudre de fer,
b) de 3 % à 25 % d'un matériau carboné constitué de carbone activé, de carbone non-activé, ou de mélanges de ceux-ci,
c) de 0,5 % à 10 % d'un sel métallique, et
d) de 1 % à 40 % d'eau,
de préférence, ladite composition exothermique comporte de plus des matériaux de rétention d'eau supplémentaires, des liants secs, des auxiliaires d'agglomération, ou des mélanges de ceux-ci, de manière plus préférée dans lequel ladite composition exothermique est sous forme de granules agglomérées.

7. Dilatateur nasal (10) selon la revendication 1, dans lequel ledit second côté (25) de ladite bande de matériau de base flexible (21) et ledit second côté (39) de ladite bande de matériau supérieur flexible (37) sont formés d'un film perméable à l'oxygène et sont rendus perméables à l'oxygène en perforant au moins une bande parmi ladite bande de matériau de base flexible (21) et ladite bande de matériau supérieur flexible (37) à l'aide d'au moins une broche, de préférence de 20 à 60 broches, pour former au moins un trou d'aération, de préférence une pluralité de trous d'aération, ayant un diamètre de 0,2 mm à 2 mm, de préférence de 0,4 mm à 0,9 mm.

8. Dilatateur nasal (10) selon l'une quelconque des revendications précédentes, comportant de plus des première (27a) et seconde (27b) garnitures de libération recouvrant la substance adhésive (26) sur ladite première région d'extrémité (22a) et ladite seconde région d'extrémité (22b) dudit premier côté (24) de ladite bande de matériau de base flexible (21), de préférence recouvrant la substance adhésive (26) sur ladite première région d'extrémité (22a), ladite seconde région d'extrémité (22b) et un segment intermédiaire (23) dudit premier côté (24) de ladite bande de matériau de base flexible (21), dans lequel lesdites première et seconde garnitures de libération sont facilement amovibles depuis ladite bande de matériau de base flexible (21) pour exposer ladite substance adhésive (26) et permettre audit élément de bandage unitaire (20) d'être fixé audit tissu de paroi extérieure (43, 44) dudit premier passage nasal (45) et dudit second passage nasal (46).

9. Dilatateur nasal (10) selon l'une quelconque des revendications précédentes, dans lequel ledit premier côté (24) de ladite bande de matériau de base flexible (21) comporte un agent thérapeutique ou actif pharmaceutique à délivrer à travers la peau du nez, de préférence dans lequel une couche de substrat séparée comportant un agent thérapeutique ou actif pharmaceutique à délivrer à travers la peau du nez est ajoutée entre ladite bande de matériau de base flexible (21) et ladite couche de substance adhésive (26), de manière plus préférée dans lequel ledit agent thérapeutique ou actif pharmaceutique comporte des composés aromatiques actifs, des composés aromatiques non-actifs, des décongestionnants, des agents antitussifs, des anti-histamines, des antibiotiques, des vitamines, des agents antiviraux, des analgésiques, des agents anti-inflammatoires incluant des agents anti-inflammatoires non-stéroïdiens, des antipruritiques, des antipyrétiques, des agents anesthésiques, des antifongiques, des antimicrobiens, ou des mélanges de ceux-ci.

10. Dilatateur nasal (10) selon l'une quelconque des revendications précédentes, comportant de plus un emballage imperméable à l'air enfermant ledit dilatateur nasal, dans lequel ledit dilatateur nasal reste étanche à l'intérieur dudit emballage imperméable à l'air jusqu'à ce qu'un utilisateur soit prêt à appliquer le dilatateur nasal sur le nez dudit utilisateur, de sorte que l'ouverture dudit emballage imperméable à l'air permet à l'oxygène provenant de l'air ambiant d'activer ledit élément chauffant pour générer un chauffage commandé et prolongé.
